(19) Europäisches Patentamt
European Patent Office
Office européen des brevets

(11) Publication number: 0 257 890
A2

(12) # EUROPEAN PATENT APPLICATION

(21) Application number: 87307023.9

(22) Date of filing: 07.08.87

(51) Int. Cl.⁴: **A61K 45/02**, A61K 37/02, //A61K45:02,A61K31:02

The applicant has filed a statement in accordance with Rule 28 (4) EPC (issue of a sample only to an expert). Accession number(s) of the deposit(s): FERM BP - 628; FERM BP - 852.

(30) Priority: 13.08.86 JP 190829/86
02.09.86 JP 207453/86
23.06.87 JP 157288/87

(43) Date of publication of application: 02.03.88 Bulletin 88/09

(84) Designated Contracting States: BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Ootsu, Koichiro
9-26 Todaiji 3-chome Shimamoto-cho
Mishima-gun Osaka 618(JP)

(74) Representative: Laredo, Jack Joseph et al
Elkington and Fife High Holborn House 52/54
High Holborn
London, WC1V 6SH(GB)

(54) **Antitumor agent.**

(57) A combined use of an interleukin-2 active substance with an interferon-α active substance by intramuscular administration exhibits a remarkably more potent antitumor activity than the single use of the respective active substances.

Fig. 1

```
1
Ala Pro Thr Ser Ser Ser Thr Lys Lys Thr Gln Leu Gln
                        20
Leu Glu His Leu Leu Leu Asp Leu Gln Met Ile Leu Asn

Gly Ile Asn Asn Tyr Lys Asn Pro Lys Leu Thr Arg Met
40
Leu Thr Phe Lys Phe Tyr Met Pro Lys Lys Ala Thr Glu
                            60
Leu Lys His Leu Gln Cys Leu Glu Glu Glu Leu Lys Pro

Leu Glu Glu Val Leu Asn Leu Ala Gln Ser Lys Asn Phe
    80
His Leu Arg Pro Arg Asp Leu Ile Ser Asn Ile Asn Val
                                100
Ile Val Leu Glu Leu Lys Gly Ser Glu Thr Thr Phe Met

Cys Glu Tyr Ala Asp Glu Thr Ala Thr Ile Val Glu Phe
        120
Leu Asn Arg Trp Ile Thr Phe Cys Gln Ser Ile Ile Ser
        133
Thr Leu Thr
```

EP 0 257 890 A2

# ANTITUMOR AGENT

The present invention relates to an antitumor agent which comprises an interleukin-2 active substance in combination with an interferon-α active substance, wherein the administration route is intramuscular injection. Said agent may further comprise a muramyldipeptide.

Attempts have recently been made to use lymphokines such as interleukin-2 (hereinafter also briefly referred to as "IL-2") for the treatment of malignant tumors in humans [Gan To Kagaku Ryoho (Japanese Journal of Cancer and Chemotherapy), *13* , 977 (1986)]. A type of IL-2 obtained via genetic engineering technique (hereinafter also briefly referred to as "rIL-2") has recently become known [Japanese Patent Laid-open No. 60-115528 which correspond to European Patent Publication No. 145390; Japanese Patent Laid-open No. 61-78799 which corresponds to European Patent Publication No. 176299].

On the other hand, interferon-α (hereinafter also briefly referred to as "IFN-α") has recently been found to possess antitumor activity; even a type of interferon-α obtained via genetic engineering technique (hereinafter also briefly referred to as "rIFN-α") is reported to exhibit a clinically significant antitumor effect [Shokaki Geka Seminar (Digestive Organ Surgery Seminar), *16*, 45 (1984)]. Specifically, rIFN-αA/D [subspecies resulting from the combination of the amino-terminal fragment between the 1st and 62nd positions of rIFN-αA and the carboxyl-terminal fragment between the 64th and 166th positions of rIFN-αD], which is a novel human IFN-α obtained by introducing into *Escherichia coli* a gene artificially synthesized from rIFN-αA [Japanese Patent Laid-open No. 57-79897 which corresponds to European Patent Publication No. 43980] and rIFN-αD, was found to exhibit bioactivity on mice also without interspecific specificity [Journal of Biological Chemistry, *257*, 11497-11502 (1982)]. Since then, it has been possible to use mice to evaluate the bioactivity of human interferon-α; its action on various immune systems have been confirmed.

In addition, a therapeutic or preventive drug for immunological diseases composed of human interleukin-2 and α-interferon is disclosed in Japanese Patent Laid-open No. 58-157723 which corresponds to European Patent Publication No. 89062, but which discloses no action other than an enhancing effect on *in vitro* natural killer cell activity, still it discloses nothing on an antitumor effect via a specific administration route.

Furthermore, it is disclosed that a muramyldipeptide is of value as an anti-tumor agent [U.S. Patent No. 4,369,178.].

Attempts have been made to employ each of the above-mentioned interleukin-2 and interferon-α for tumor treatment, and it is known that human interleukin-2 and interferon-α are used as therapeutic or preventive drugs for immunological diseases. The purpose of the present invention is to provide an antitumor agent which comprises interleukin-2 in combination with interferon-α, wherein the administration route is intramuscular injection; the present antitumor agent exhibits a remarkably strong antitumor effect due to the said administration route. Furthermore, the purpose of the present invention is to provide an antitumor agent which comprises interleukin-2 in combination with interferon-α and a muramyldipeptide. This antitumor agent brings a remarkable antitumor activity at a lower dose level.

The present inventor found that IL-2 and IFN-α, when administered in combination to animals by intramuscular injection, exhibit a remarkably strong antitumor effect even at a low dose level, and that further combination with a muramyldipeptide by intramuscular injection brings a remarkable antitumor activity at a lower dose level. The present inventor made further intensive research based on this finding and completed the present invention.

The present invention is directed to (1) a method for treating or improving a tumor disease of a warm-blooded animal which comprises administering by intramuscular injection an interleukin-2 active substance in combination with an interferon-α active substance to the warm-blooded animal. In said method, a muramyldipeptide may be also administered by intramuscular injection.

The present invention is also directed to (2) use of an interleukin-2 active substance in combination with an interferon-α active substance for the production of a pharmaceutical preparation, wherein the administration route is intramuscular injection for treating or improving a tumor disease of a warm-blooded animal. In said use, a muramyldipeptide may be further employed.

The present invention furthermore is directed to (3) an antitumor agent which comprises an interleukin-2 active substance in combination with an interferon-α active substance, wherein the administration route is intramuscular injection. Said agent may further comprise a muramyldipeptide.

Any substance can be used as interleukin-2 active substance for the above purpose, as long as it possesses IL-2 activity, namely, the activity enabling the subcultivation of T-cells while maintaining their functions.

As examples of such substances, mention may be made of natural IL-2 produced in animal bodies or by animal cells, IL-2 produced via genetic engineering technique and related substances. It does not matter whether or not said IL-2 species or related substances, when they are protein, have a sugar chain.

Specifically, interleukin-2 active substances which can be used include the polypeptide (I) (human IL-2) having the amino acid sequence shown in Figure 1 and fragments comprising a partial amino acid sequence essential to the biological or immunological activity of the polypeptide. As examples of such fragments, mention may be made of fragments resulting from the deletion of either 1 amino-terminal amino acid residue (European Patent Publication No. 91539) or 4 amino-terminal amino acid residues (Japanese Patent Laid-open No. 60-126088) from the polypeptide (I) and fragments resulting from the deletion of several carboxyl-terminal amino acid residues from the polypeptide (I). It is also possible to use fragments resulting from either the deletion of substitution of some amino acid residues in the polypeptide (I), for example, a fragment resulting from the substitution of the cysteine residue at the 125th position by a serine residue [Japanese Patent Laid-open No.59-93093 which corresponds to U.S. Patent No. 4,518,584.].

The above IL-2 active substances may be chemically modified with polyethylene glycol derivatives or the like [e.g. Japanese Patent Laid-open No. 60-226821].

It is especially preferable that the human IL-2 having the amino acid sequence shown in Figure 1 be used for the present invention. In this case, a mixture of the relevant human IL-2 having an additional methionine residue ( *Met*) at its amino terminal and that having no additional methionine residue [e.g. Japanese Patent Laid-open No. 60-115528 which corresponds to European Patent Publication No. 145390; Japanese Patent Laid-open No. 61-78799 which corresponds to European Patent Publication No. 176299.] can also be used, and the relevant human IL-2 may have no Met at the amino-terminal and have an alanine residue (*Ala*) as the starting amino acid residue [e.g. Japanese Patent Laid-open No.61-78799 which corresponds to European Patent Publication No. 176299.].

Any substance can be used as interferon-α active substance, as long as it possesses IFN-α activity, namely an antiviral activity; any type of interferon-α can be used, whether it be a natural-type interferon-α or an interferon-α obtained via genetic engineering technique. As examples of IFN-α obtained via genetic engineering technique, mention may be made of rIFN-αA, B, C, D, E, F, G, H, I and J [Japanese Patent Laid-open No. 57-79897 which corresponds to European Patent Publication No. 43980], rIFN-αA/D [subspecies resulting from the combination of the amino-terminal fragment between the 1st and 62nd positions of rIFN-αA and the carboxyl-terminal fragment between the 64th and 166th positions of rIFN-αD] [Journal of Biological Chemistry, 257, 11497-11502 (1982)] and rIFN-αD/A and rIFN-αA/D/A as described in the said literature.

As the muramyldipeptide, there may be mentioned compounds of the formula (II)

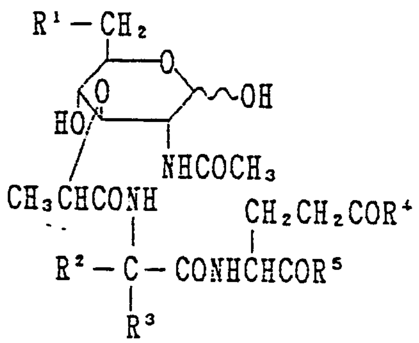

(II)

wherein $R^1$ is a hydroxyl group or a carboxylic acid residue, $R^2$ and $R^3$ each independently is hydrogen or a lower ($C_{1-6}$) alkyl group, which may optionally be substituted by a hydroxyl group, $R^4$ is a hydroxyl group or a lower ($C_{1-6}$) alkoxy group and $R^5$ is a hydroxyl group or a substituted or unsubstituted amino group, and physiologically acceptable salts thereof.

The compounds of formula (I) and salts thereof are known compounds and are described in United States Patent No. 4,101,536, Japanese Patent Laid-open No. 54-63016, Japanese Patent Laid-open No. 54-79228 (which corresponds to European Patent Publication No. 2677) and Japanese Patent Laid-open No. 55-111499 (which corresponds to U.S. Patent No. 4,369,178), for instance, and they can be produced by the method described in said literature references.

Thus, referring to the formula (II), the carboxylic acid residue represented by $R^1$ is, for example, a $C_2$-$C_{50}$ carboxylic acid residue such as mycoloyl, stearoyl, oleoyl and a group of the formula (III)

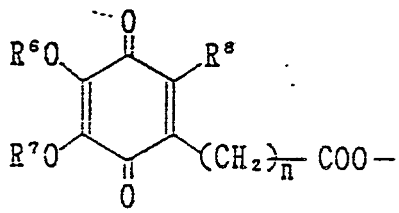

(III)

wherein $R^6$, $R^7$ and $R^8$ each independently is a lower ($C_{1-4}$) alkyl and n is an integer of 1 to 10, inclusive. Such carboxylic acid residue may further contain an intervening amino acid residue such as a glycine, alanine or β-alanine residue. The lower alkyl group represented by $R^2$ and/or $R^3$ is preferably a $C_1$-$C_4$ alkyl, for example methyl, ethyl, propyl, isopropyl, butyl, isobutyl, secbutyl or t-butyl. When substituted by a hydroxyl group, such lower alkyl is mentioned as hydroxymethyl or 2-hydroxyethyl, for instance.

The lower alkoxy represented by $R^4$ is preferably a $C_1$-$C_3$ alkoxy, namely methoxy, ethoxy, propoxy or isopropoxy. The hydroxyl group represented by $R^4$ may have a hapten-active substituent such as an ester residue of N-hydroxy-5-norbornene-2,3-dicarboxyimide.

The substituted or unsubstituted amino group represented by $R^5$ is a primary amino group or an amino group having one or two substituents. Examples of said substituents are lower ($C_1$-$C_3$) alkyl (e.g. methyl, ethyl, propyl, isopropyl, etc.), phenyl and aralkyl (e.g. benzyl, phenethyl, etc.).

As the preferred embodiment of the muramyldipeptide, there may be mentioned the compound of the formula (II), in which $R^1$ is a hydroxyl group or a $C_2$-$C_{50}$ carboxylic acid residue of the formula (III) wherein $R^6$, $R^7$ and $R^8$ each independently is a lower ($C_{1-4}$) alkyl and n is an integer of 1 to 10, $R^2$ and $R^3$ is each independently hydrogen or a lower ($C_{1-6}$) alkyl group which may optionally be substituted by a hydroxyl group, $R^4$ is a hydroxyl group or a lower ($C_{1-6}$) alkoxy group, and $R^5$ is a hydroxyl group or an amino group.

Typical examples of the muramyldipeptide of the above formula (I) are muramyldipeptide mycolic acid esters (e.g. 6-0-mycomycoloyl-N-acetylmuramyl-L-alanyl-D-isoglutamine, 6-0-mycomycoloyl-N-acetylmuramyl-L-seryl-D-isoglutamine, 6-0-nocardomycoloyl-N-acetyl-muramyl-L-seryl-D-isoglutamine, 6-0-ursomycoloyl-N-acetylmuramyl-L-seryl-D-isoglutamine), muramyldipeptide fatty acid esters (e.g. 6-0-stearoyl-N-acetylmuramyl-L-alanyl-D-isoglutamine, 6-0-stearoyl-N-acetylmuramyl-L-seryl-D-isoglutamine, 6-0-oleoyl-N-acetylmuramyl-L-alanyl-D-isoglutamine), muramyldipeptide quinonylalkanoic acid esters (e.g. 6-0-[3-(2,3-dimethoxy-5-methyl-1,4-benzoquinon-6-yl)propionyl]-N-acetylmuramyl-L-valyl-D-isoglutamine, 6-0-[10-(2,3-dimethoxy-5-methyl-1,4-benzoquinon-6-yl)decanoyl]-N-acetylmuramyl-L-valyl-D-isoglutamine, 6-0-[10-(2,3-dimethoxy-5-methyl-1,4-benzoquionon-6-yl)decanoyl]-N-acetylmuramyl-L-seryl-D-isoglutamine, muramyldipeptides (e.g. N-acetylmuramyl-L-alanyl-D-isoglutamine (abbreviated as TMD-1)), N-acetylmuramylaminoisobutyryl-D-isoglutamine (abbreviated as TMD-5) and lower alkyl esters in the isoglutamine moiety of the above compounds (e.g. 6-0-[3-(2,3-dimethoxy-5-methyl-1,4-benzoquinon-6-yl)-propionyl]-N-acetylmuramyl-L-valyl-D-isoglutamine methyl ester (abbreviated as TMD-76), 6-0-[10-(2,3-dimethoxy-5-methyl-1, 4-benzoquinon-6-yl)decanoyl]-N-acetylmuramyl-L-valyl-D-isoglutamine methyl ester (abbreviated as quinonyl-MDP-66), 6-0-[10-(2,3-dimethoxy-5-methyl-1,4-benzoquinon-6-yl)decanoyl]-N-acetylmuramyl-L-seryl-D-isoglutamine methyl ester.

In the practice of the present invention, TMD-1, TMD-5, TMD-76, quinonyl-MDP-66 and the like water-soluble muramyldipeptides are particularly preferred among the above-mentioned muramyldipeptides.

It is desirable that the specific activity of the IL-2 active substance be about 20,000 to 80,000 unit/mg. Aqueous solutions of IL-2 active substances, whose activity is about 1 to 80,000 unit/mℓ, can work well; the use of solutions whose specific activity is about 10 to 50,000 unit/mℓ is particularly suitable.

In addition, it is desirable that the specific activity of the IFN-α active substance be about 100,000,000 to 1,000,000,000 unit/mg. Aqueous solutions of IFN-α active substances, whose activity is about 10,000,000 to 1,000,000,000 unit/mℓ, can work well; the use of solutions whose activity is about 5,000,000 to 500,000,000 unit/mℓ is particularly suitable.

It is recommended that the ratio of IL-2 active substance and IFN-α active substance used in the present antitumor agent by unit be about 10 to 3000 units to about 8 X $10^3$ X $10^9$ units, preferably about 100 to 2,000 units to about 8 X $10^4$ to 8 X $10^6$ units, more preferably about 500 to 2,000 units to about 8 X $10^5$ to 8 X $10^6$ units.

The amounts of IL-2 active substance and interferon-α active substance used for the present invention vary with application method, purpose and the like. It is desirable that interferon-α be used in a ratio of about 500,000 to 500,000,000 units to 1 mcg of the protein of IL-2 active substance [35 units, calculated on the IL-2 activity basis; for information about the measurement of IL-2 activity, refer to Japanese Patent Laid-open No. 60-115528 which corresponds to European Patent Publication No. 145390], and the use of a ratio

of about 500,000 to 100,000,000 units [for information about the measurement of IFN-α activity, refer to Japanese Patent Laid-open No. 57-79897 which corresponds to European Patent Publication No. 43980] to 1 mcg of the protein of IL-2 active substance. As described above, the interleukin-2 active substance and interferon-α active substance used in the present invention can work well even at a low dose level, for example, about 5 to 500 mcg/kg/day, calculated on the total protein amount basis, for mouse and about 0.01 to 20 mcg/kg/day, calculated on the total protein amount basis, for mammals such as cat, dog, bovine, horse, sheep, goat, rabbit and human

The daily dose of IL-2 active substance is preferably about 1 mcg/kg to 500 mcg/kg for mouse, and about 0.005 mcg/kg to 10 mcg/kg for mammals such as cat, dog, bovine, horse, sheep, goat, rabbit and human.

The daily dose of IFN-α active substance is preferably about 3 mcg/kg to 2000 mcg/kg for mouse, and about 0.005 mcg/kg to 50 mcg/kg for mammals such as cat, dog, bovine, horse, sheep, goat, rabbit and human.

The amount of the present antitumor agent, which comprises IL-2 active substance in combination with IFN-α active substance and a muramyldipeptide, vary with application method, purpose and the like. The ratio of the IL-2 active substance: the IFN-α active substance: the muramyldipeptide is preferably about 10 to 3000 units: 8 X $10^3$ to 8 X$10^9$ units: 1 mcg to 2,000 mcg, more preferably about 100 to 2000 units: 8 X $10^4$ to 8 X $10^6$ units: 5 mcg to 1000 mcg.

The present antitumor agent, which comprises IL-2 active substance in combination with IFN-α active substance and a muramyldipeptide, can work well even at a lower dose level, for example, about 0.5 to 50 mcg/kg/day for mouse, and about 0.001 to 2 mcg/kg/day for mammals such as cat, dog, bovine, horse, sheep, goat, rabbit and human.

The daily dose of IL-2 active substance is preferably about 0.1 mcg/kg to 50 mcg/kg for mouse, and about 0.0005 mcg/kg for mammals such as cat, dog, bovine, horse, sheep, goat, rabbit and human.

The daily dose of IFN-α active substance is preferably about 0.3 mcg/kg to 200 mcg/kg for mouse, and about 0.0005 mcg/kg to 5 mcg/kg for mammals such as cat, dog, bovine, horse, sheep, goat, rabbit and human.

The daily dose of the muramyldipeptide is preferably about 50 mcg/kg to 1000 mcg/kg for mouse, and about 0.001 mcg/kg to 5 mcg/kg for mammals such as cat, dog, bovine, horse, sheep, goat, rabbit and human.

The IL-2 active substance and IFN-α active substance of the present invention are both low in toxicity. For example, the intravenous injection of the IL-2 of a mixture of IL-2 having an amino acid sequence shown in Fig. 1 and IL-2 having an amino acid sequence shown in Fig. 1 and Met at the N-terminus, and which is obtained by the purification method described in Japanese Patent Laid-open No. 60-115528 which corresponds to European Patent Publication No. 145,390 at a dosage level of 10 mg/kg (1mg = 3.5 X $10^4$ units) causes no death due to toxicity in mice or rats. As to the toxicity of IFN-α active substances, neither mouse nor rat dies even after 1 week of consecutive administration of 1 X $10^7$ units of rIFN-α A/D, which is known to exhibit activity on mice and rats. As stated above, both substances are low in toxicity, and they can be thus safely administered to mammals.

Furthermore, the minimum lethal doses (MLDs) for the muramyldipeptide is not less than 500 mg/kg (s.c.) in rats. Therefore, the muramyldipeptide can be used safely.

The antitumor agent for intramuscular injection of the present invention is a combination of an interleukin-2 active substance and an interferon-α active substance. Furthermore, it may have a combination of a muramyldipeptide.

Forms of the present antitumor agent may include aqueous solutions of the respective substances, solid mixtures prepared by lyophilizing the respective substances, respective solid products prepared by lyophilizing aqueous solutions of each substance and combinations of an aqueous solution of one substance and a solid product prepared by lyophilizing the other substance.

The antitumor agent of the present invention can be administered in the form of a single preparation prepared by mixing the said substances with pharmaceutically acceptable diluents, excipients, etc. in accordance with pharmaceutical production methods known per se if desired. The base substances can be separately prepared as individual drugs using pharmaceutically acceptable diluents, excipients etc. if desired, after which they can be administered as a single preparation prepared using a diluent etc. before use. It is also possible to individually administer such separately prepared drugs to the same subject either simultaneously or at an interval.

Aqueous solutions of the present antitumor agent can be prepared via conventional methods using solvents such as aqueous solvents (e.g. distilled water), water-soluble solvents (e.g. physiological saline solution and Ringer solution) and oily solvents (e.g. sesame oil and olive oil) or if desired using additives such as solubilizers (e.g. sodium salicylate and sodium acetate), buffers (e.g. sodium citrate and glycerol), isotonizing agents (e.g. glucose and invert sugars), stabilizing agents (e.g. human serum albumin and polyethylene glycol), preservatives (e.g. benzyl alcohol and phenol) and pain-reducing agents (e.g. benzalkonium chloride and procaine hydrochloride).

The concentration of IL-2 active substance in the said aqueous solution is preferably about 0.3 to 1,000 mg/ml, more preferably about 3 to 500 mg/.ml.

The concentration of IFN-α active substance in the said aqueous solution is preferably about 0.01 to 100 mg/ml, more preferably about 0.5 to 50 mg/ml.

The concentration of the muramyldipeptide in the said aqueous solution is preferably about 0.02 to 100 mg/ml, more preferably about 0.2. to 100 mg/ml.

The said aqueous solutions are adjusted to a pH of about 3 to 7, preferably about 5 to 7. The pH adjustment to the above range is achieved by adding, for example, dilute hydrochloric acid or dilute alkali (e.g. dilute sodium hydroxide, dilute sodium bicarbonate).

Among the preparation of the present antitumor agent, solid ones can be prepared as solid drugs for intramuscular injection, for example, by lyophilizing the above-mentioned aqueous solution or mixing a solid (e.g. powdery) substance of the respective ingredient with diluents (e.g. distilled water, physiological saline solution and glucose), excipients (e.g. carboxymethylcellulose (CMC) and sodium arginate), preservatives (e.g. benzyl alcohol, benzalkonium chloride and phenol), pain-reducing agents (e.g. glucose, calcium gluconate and procaine hydrochloride) etc. via conventional means.

It is favorable that the solution which contains IL-2 active substance and/or IFN-α active substance is adjusted so that its solution exhibits a pH of about 3 to 7 by adding human serum albumin (hereinafter also briefly referred to as HSA), because the reduction of IL-2 and IFN-α activities during storage, freezing or lyophilization process is lessened, and a clear solution is obtained from lyophilized product at the time of re-dissolution.

Any HSA can be used, but the use of HSA of such quality that it is used for parenteral administration is preferable for the clinical application of the present composition.

For example, HSA obtained by fractionating and purifying the starting material, normal human blood plasma, via Cohn's methanol fractionation method 6 can be used.

It is recommended that HSA be contained at a concentration of about 0.1 to 50 mg, preferably about 0.5 to 20 mg per each mℓ of aqueous solution of the respective ingredient.

The preparation of the present antitumor agent may be produced by mixing with not only the above HSA but also either 1 or not less than 2 of amino acids such as glycine, glutamic acid, aspartic acid, alanine and proline, specifically monoamino aliphatic or cyclic amino acids; monosaccharides such as glucose and mannose; sugar alcohols such as sorbitol and mannitol; and physiologically acceptable salts or derivatives of these substances.

In cases where the preparation is in aqueous solution, it is preferable that the above-mentioned mixing substances be added in a ratio of about 10 to 100 mg (for monosaccharides or sugar alcohols) and about 5 to 50 mg (for amino acids) to 1 mℓ of the solution.

When an acidic amino acid such as glutamic acid is added to adjust the the preparation (mixed with HSA) of the present invention in solution to a pH of about 3 to 7, preferably about 3 to 5.5, the specified pH value can be obtained by adding the said substance in an amount as specified above. If desired or when said acidic amino acid is not added, the present preparation is adjusted to the specified pH with a mineral acid such as hydrochloric acid or phosphoric acid or buffering agent such as succinic acid, tartaric acid or citric acid.

It is preferable that the antitumor agent of the present invention take the form of an aqueous solution, frozen product or lyophilized product, of which lyophilized product is particularly suitable.

The antitumor agent of the present invention can be produced via, for example, the following method for the prevention of the attenuation of the IL-2 active substance.

To an aqueous solution containing about 1 to 800000 unit/mℓ preferably about 1 to 80,000 unit/mℓ of the IL-2 active substance and about 10,000,000 to 1,000,000,000 unit/mℓ of the IFN-α active substance, and when a muramyldipeptide is contained, the aqueous solution containing about 1 to 200 mg of the muramyldipeptide, HSA is added to a concentration as specified above, if desired, then pH adjustment is carried out via the above-mentioned method.

Monosaccharides, sugar alcohols, amino acids etc. may also be added to a concentration as specified above for each substance, if desired. Isotonizing agents, surfactants etc. may also be added, if desired. In cases where substances other than HSA are added, pH adjustment is carried out via the above-mentioned method so that the finally obtained aqueous solution exhibits a pH value as specified above. The antitumor agent of the present invention in aqueous solution thus obtained can also be used as a starting material for the frozen and lyophilized products described below.

The antitumor agent of the present invention in the form of a frozen product can be produced, for example, by freezing the above aqueous solution usually at about - 80 to -20°C. It is preferable that the frozen composition thus obtained be stored at about - 80 to -10°C.

The antitumor agent of the present invention in the form of a lyophilized product can be produced, for example, by drying the above-mentioned frozen composition under reduced pressure via a conventional method or drying under reduced pressure via a conventional method either the above aqueous solution or an aqueous solution obtained by thawing the above-mentioned frozen composition after subdividing (if desired) and freezing in the same manner as above.

In addition, the antitumor agent of the present invention in solution can be produced also by re-dissolving the lyophilized product prepared via the above-mentioned method in a solution which contains, for example, the above-mentioned monosaccharides, sugar alcohols and amino acids and which is pre-adjusted to the specified pH value with hydrochloric acid etc. if desired.

When the antitumor agent of the present invention is produced in the form of a lyophilized drug for injection, it is preferable that an aqueous solution of the respective ingredient and an aqueous solution containing additives, after mixing together after filtration for bacterial removal, or a mixed solution of these aqueous solutions, after pipetting into vials etc. via an aseptic procedure after purification by filtration for bacterial removal etc., be subjected to the above-mentioned lyophilization treatment. In this case, the stability of the said composition can be improved by making the inside space of the container vacuum or replacing the inside atmosphere with nitrogen gas.

When the lyophilized product is dissolved in an aqueous solution containing amino acids, monosaccharides or sugar alcohols, it is preferable that the aqueous solution be filtered for bacterial removal, pipetted into ampules etc. via an aseptic procedure, then sterilized with steam via a conventional method before use.

The antitumor agent of the present invention is useful in treating or improving tumor disease of a warm-blooded animal such as mammals (e.g. mouse, cat, dog, bovine, horse, sheep, goat, rabbit and human); for example, it noticeably lengthens the life of mammals with tumors. As examples of target diseases, mention may be made of various leukemias, malignant lymphoma, osteosarcoma, malignant melanoma, malignant chorioephithelioma, myosarcoma, ovarian cancer, uterine cancer, prostatic cancer, pancreatic cancer, digestive system cancers such as gastric and intestinal cancers, lung cancer, esophageal cancer, neck-head tumors and brain tumors.

When the preparation of the present antitumor agent is in the form of an aqueous solution, it is directly administered as a solution for injection.

When the said composition is in the form of a lyophilized solid, it is dissolved in distilled water or physiological saline solution to prepare a solution for injection before use. If desired, the said composition can also be used after dissolving in a solution which contains saccharides, sugar alcohols, amino acids etc. similar to those mentioned above and which is pre-adjusted to the specified pH in the same manner as above.

In the antitumor agent of the present invention, individual drugs separately prepared from the IL-2 active substance, IFN-α active substances and a muramyldipeptide can be simultaneously administered to the same subject; they can also be separately administered to the same subject at an interval. An interval of, for example, about 12 to 24 hours is allowable, but the use of an interval of about 3 to 9 hours, preferable about 1 hour or less is recommended.

The antitumor agent for intramuscular injection of the present invention exhibits remarkable antitumor action as is obvious in the experimental example and comparative examples shown below, its antitumor action is much stronger in intramuscular injection than in other injection means such as subcutaneous and intravenous administrations. In addition, it exhibits strong antitumor action even when its constituent substances are used in combination at low doses.

The preparation of the present invention and HSA is characterized by excellent features; for example, it is less susceptible to the reduction of IL-2 and interferon-α activities during storage and freezing or lyophilizing procedure, and its lyophilized product provides a clear solution at the time of re-dissolution.

The preparation of the present invention and HSA, specifically its lyophilized product not only exhibits a better appearance but also is conducive to the prevention of its adsorption to container walls.

The preparation (which contains HSA) of the present antitumor agent when lyophilized, not only exhibits a better appearance but also is conducive to the alleviation of pain at the time of injection. The composition additionally containing monosaccharides and/or sugar alcohols is also conducive to the alleviation of pain at the time of injection.

The antitumor agent comprising a combination of an IL-2 active substance and an IFN-α active substance of the present invention, when administered by intramuscular injection, exhibits antitumor action much stronger than the case of other injection routes. And therefore, it is thus useful in treating or improving tumor disease of a warm-blooded animal.

Furthermore, the present antitumor agent which comprises IL-2 active substance in combination with IFN-α active substance and a muramyldipeptide brings a remarkable antitumor activity at a lower dose level.

Brief description of the drawing:

Fig. 1 shows an example of the amino acid sequence of IL-2 to be used in the practice of the present invention.

The following Experimental Examples, Comparative Examples and Examples are further illustrative of the present invention, but by no means limitative thereof.

The IL-2 is produced by the manner described in Japanese Patent Laid-open No. 60-115528 which corresponds to European Patent Publication No. 145390 or the manner described in Japanese Patent Laid-open No. 61-78799 which corresponds to European Patent Publication No. 176299, by employing a transformant Escherichia coli DH1/pTF 4 (IFO 14299, FERM BP-628) or Escherichia coli N4830/pTB 285 (IFO 14437, FERM BP-852).

The rIL-2 employed in the Experimental Examples and Comparative Examples is a mixture of a human IL-2 which has the amino acid sequence shown in Fig. 1 and whose amino terminus amino acid is Ala-(IL-2 Ala species) and an IL-2 which has the amino acid sequence shown in Fig. 1 and methionine at the amino terminus (IL-2 Met species) and the mixture of IL-2 Ala species and IL-2 Met species is produced by the above manner.

The IL-2 whose N-terminus amino acid is alanine (IL-2 Ala species) which is employed in the Examples is produced by the separation and purification procedure described in Japanese Patent Laid-open No. 61-78799 which corresponds to EPC Patent Publication No. 176299 from said mixture of IL-2 Ala species and IL-2 Met species.

The transformant Escherichia coli DH1/pTF4 has been deposited at Institute for Fermentation, Osaka (IFO), Japan under the accession number of IFO 14299 since November 25, 1983. And the transformant has been also deposited at Fermentation Research Institute, Agency of Industrial Science and Technology, Ministry of International Trade and Industry (FRI), Japan under the accession number of FERM P-7578 since April 6, 1984, and the deposit has been converted to the deposit under the Budapest Treaty and the transformant has been stored at FRI under the accession number of FERM BP-628.

The transformant Escherichia coli N4830/pTB285 has been deposited at the IFO under the accession number of IFO 14437 since April 25, 1985. And the transformant has been also deposited at theFRI under the accession number of FERM P-8199 since April 30, 1985, and the deposit has been converted to the deposit under the Budapest Treaty and the transformant has been stored at FRI under the accession number of FERM BP-852.

The IL-2s which are employed in the Experimental Examples, Comparative Examples and Examples have a specific activity of about 3.5 X $10^4$ units/mg.

The IFN-α used in the Experimental Example, Comparative Examples or Examples is produced by the method disclosed in Japanese Patent Laid-open No. 57-79897 which corresponds to EPC Publication No. 43980; the IFN-αA/D used in the examples is produced by the method described in the Journal of Biological Chemistry, *257*, 11497 (1982).

## Examples

### Experimental Example 1

Antitumor Action in Intramuscular Administration:

Using injecting cylinders, 1 X $10^6$ Meth-A fibrosarcoma cells (Meth-A tumor cells) were subcutaneously transplanted to the flank of female BALB/c mice weighing about 20 g. On the 7th day following the tumor transplantation, mice having a tumor reaching the specified level in size were selected and grouped, and drug administration was initiated. Each drug was intramuscularly administered at the left thigh once daily for 10 consecutive days. Each drug was dissolved in a physiological saline solution (solvent) supplemented with 5% normal mouse serum and prepared in a form of a single preparation so that the dose level is 0.1 mℓ/ mouse, calculated on the administration liquid volume basis. The evaluation of antitumor effects was conducted by measuring tumor weight on the 21st day following the tumor transplantation, calculating the average tumor weight for each experimental group, and calculating the tumor weight ratio (T/C %) of each drug-treated group (T, 5 mice in each group) to the non-treated control group (C, 10 mice in the group). Daily dose levels are shown in either drug weight (mcg) or interferon activity (unit) per mouse. The results of administrations of IL-2 alone, IFN-α A/D alone and the antitumor agent comprising a combination of IL-2 and IFN-α of the present invention are shown in Table 1.

Table 1

| Drug | Dose Level rIFN-αA/D (units/mouse) | Dose Level rIL-2 (mcg/mouse) | Number of Animals | Tumor Weight (mg) Mean ± SD | Tumor Weight Ratio (T/C %) | Body Weight Gain (g) (7th day - 21st day) |
|---|---|---|---|---|---|---|
| Non-treated control | | | 10 | 6,496 ± 1,017 | | 5.0 |
| Solvent (control) | | | 5 | 5,510 ± 988 | 85 | 3.9 |
| rIFN-αA/D | $0.5 \times 10^5$ | | 5 | 2,930 ± 540 | 45 | 1.4 |
| rIFN-αA/D | $1 \times 10^5$ | | 5 | 1,675 ± 496 | 26 | 0.7 |
| rIL-2 | | 5 | 5 | 2,280 ± 414 | 35 | 1.0 |
| rIL-2 | | 10 | 5 | 1,998 ± 786 | 31 | 1.4 |
| rIL-2 | | 25 | 5 | 1,738 ± 823 | 27 | 0.8 |
| rIFN-αA/D | $1 \times 10^5$ | 1 | 5 | 548 ± 242 | 8 | 0.2 |
| + | $1 \times 10^5$ | 5 | 5 | 121 ± 46 | 2 | −0.4 |
| rIL-2 | $1 \times 10^5$ | 10 | 5 | 145 ± 70 | 2 | −0.4 |
| | $0.5 \times 10^5$ | 10 | 5 | 139 ± 115 | 2 | −0.8 |
| | $0.25 \times 10^5$ | 10 | 5 | 108 ± 44 | 2 | 0 |

Note) SD: Standard deviation

T/C %: Mean weight for drug-treated group/mean weight for non-treated control group × 100

Comparative Example 1

Antitumor Action in Subcutaneous Administration:

Using injecting cylinders, 1 X $10^6$ Meth-A fibrosarcoma cells (Meth-A tumor cells) were subcutaneously transplanted to the flank of female BALB/c mice weighing about 20 g. On the 7th day following the tumor transplantation, mice having a tumor reaching the specified level in size were selected and grouped, and drug administration was initiated. Each drug was subcutaneously administered at the flank in the opposite side of the tumor transplantation site once daily for 10 consecutive days. Each drug was dissolved in a physiological saline solution (solvent) supplemented with 5% normal mouse serum and prepared in a form of a single preparation so that the dosage level was 0.1 mℓ/mouse , calculated on the administration liquid volume. The evaluation of antitumor effects was conducted in the same manner as in Experimental Example 1. The results are shown in Table 2.

Table 2

| Drug | Dose Level rIFN-αA/D (units/mouse) | Dose Level rIL-2 (mcg/mouse) | Number of Animals | Tumor Weight (g) Mean ± SD | Tumor Weight Ratio* (T/C %) | Body Weight Gain (g) (7th day - 21st day) |
|---|---|---|---|---|---|---|
| Non-treated control | | | 10 | 3.68±0.91 | | 2.8 |
| Solvent (control) | | | 5 | 3.75±1.01 | 102 | 2.9 |
| rIFN-αA/D | $2 \times 10^5$ | | 5 | 1.41±0.63 | 38 | −0.4 |
| rIL-2 | | 10 | 5 | 1.43±0.50 | 39 | 2.1 |
| rIFN-αA/D | $2 \times 10^5$ | 1 | 5 | 0.88±0.41 | 24 | 0.3 |
| + | $2 \times 10^5$ | 5 | 5 | 0.63±0.29 | 17 | 0 |
| rIL-2 | $2 \times 10^5$ | 10 | 5 | 0.43±0.24 | 12 | −0.5 |
| | $0.5 \times 10^5$ | 10 | 5 | 0.81±0.19 | 22 | 0.5 |
| | $1 \times 10^5$ | 10 | 5 | 0.48±0.19 | 13 | 0.3 |

*: Ratio (%) of drug-treated group (T) to non-treated control group (C)

Comparative Example 2

Antitumor Action in Intravenous Administration:

Using injecting cylinders, 1 x $10^6$ Meth-A tumor cells were subcutaneously transplanted to the flank of female BALB/c mice weighing about 20 g. On the 7th day following the tumor transplantation, mice having a tumor reaching the specified level in size were selected and grouped, and drug administration was initiated. Each drug was administered via the tail vein once daily for 10 consecutive days. Each drug was dissolved in a physiological saline solution (solvent) supplemented with 5% normal mouse serum and prepared in a form of a single preparation so that the dosage level is 0.2 mℓ/mouse, calculated on the administration liquid basis. On the 21st day following the tumor transplantation, antitumor effects were evaluated by measuring tumor weight, calculating the mean tumor weight for each experimental group, and calculating the tumor weight ratio (T/C %) of each drug-treated group (T, 5 mice in each group) to the non-treated control group (C, 10 mice in the group).

The results of administrations of IL-2 alone, IFN-α alone and the antitumor agent comprising a combination of IL-2 and IFN-α of the present invention are as shown in Table 3. Daily dosage levels are shown in either drug weight (mcg) or interferon activity (unit) per mouse.

Table 3

| Drug | Dose Level rIFN-αA/D (units/mouse) | Dose Level rIL-2 (mcg/mouse) | Number of Animals | Tumor Weight (g) Mean ± SD | Tumor Weight Ratio (T/C %) | Body Weight Gain (g) (7th day - 21st day) |
|---|---|---|---|---|---|---|
| Non-treated control | | | 15 | 3.53 ± 1.18 | | 3.0 |
| Solvent (control) | | | 5 | 2.65 ± 0.36 | 75 | 1.7 |
| rIFN-αA/D | $2 \times 10^5$ | | 5 | 1.23 ± 0.56 | 35 | 0.2 |
| rIL-2 | | 10 | 5 | 2.00 ± 0.71 | 57 | 1.3 |
| rIFN-αA/D | $2 \times 10^5$ | 1 | 5 | 0.72 ± 0.29 | 20 | −0.2 |
| + | $2 \times 10^5$ | 5 | 5 | 0.70 ± 0.12 | 20 | 0.3 |
| rIL-2 | $2 \times 10^5$ | 10 | 5 | 0.36 ± 0.16 | 10 | −0.8 |
| | $0.5 \times 10^5$ | 10 | 5 | 0.59 ± 0.16 | 17 | 0 |
| | $1 \times 10^5$ | 10 | 5 | 0.54 ± 0.13 | 15 | 0.1 |

Experimental Example 2

Antitumor Action in Intramuscular Administration of rIL-2, rIFN-α A/D and N-acetylmuramyl-L-alanyl-D-isoglutamine(TMD-1):

Using injecting cylinders, 1 X $10^6$ Meth-A fibrosarcoma cells (Meth-A tumor cells) were subcutaneously transplanted to the flank of female BALB/c mice weighing about 20 g. On the 7th day following the tumor transplantation, mice having a tumor reaching the specified level in size were selected and grouped, and drug administration was initiated. Each drug was intramuscularly administered at the left thigh once daily for 10 consecutive days. Each drug was dissolved in a physiological saline solution (solvent) supplemented with 5% normal mouse serum and prepared in a form of a single preparation so that the dosage level is 0.1 mℓ/mouse, calculated on the administration liquid volume basis. The evaluation of antitumor effects was conducted by measuring tumor weight on the 21st day following the tumor transplantation, calculating the average tumor weight for each experimental group, and calculating the tumor weight ratio (T/C %) of each drug-treated group (T, 5 mice in each group) to the non-treated control group (C, 10 mice in the group). Daily dosage levels are shown in either drug weight (mcg) or interferon activity (unit) per mouse. The results of administrations of IL-2 alone, IFN-α A/D alone and the antitumor agent comprising a combination of IL-2, IFN-α and a muramyldipeptide of the present invention are as shown in Table 4.

Table 4

| Drug | Dose Level rIFN-αA/D (units/mouse) | rIL-2 (mcg/mouse) | TMD-1 (mcg/mouse) | Number of Animals | Tumor Weight (g) Mean±SD | Tumor Weight Ratio (T/C %) | Body Weight Gain (g) (7th day - 21st day) |
|---|---|---|---|---|---|---|---|
| Non-treated control | | | | 10* | 6.67±1.54 | | 4.6 |
| Solvent (control) | | | | 5* | 6.60±2.20 | 99 | 4.1 |
| rIFN-αA/D | 2 × $10^4$ | | | 5 | 3.48±0.74 | 52 | 2.1 |
| rIL-2 | | 0.5 | | 5 | 3.94±1.08 | 59 | 3.3 |
| TMD-1 | | | 200 | 5** | 3.95±0.84 | 59 | 0.2 |
| rIFN-αA/D + rIL-2 | 2 × $10^4$ | 0.5 | | 5 | 1.81±1.12 | 27 | 1.8 |
| rIFN-αA/D + TMD-1 | 2 × $10^4$ | | 200 | 5 | 2.36±1.43 | 35 | 2.3 |
| rIL-2 + TMD-1 | | 0.5 | 200 | 5 | 2.71±1.52 | 41 | 2.2 |
| rIFN-αA/D + rIL-2 + TMD-1 | 2 × $10^4$ | 0.5 | 200 | 5 | 0.15±0.12 | 2 | 0.3 |

Experimental Example 3

Antitumor Action in Intramuscular Administration of rIL-2 in combination with IFN-αA/D and TMD-1:

Under the same conditions as in the Experimental Example 1, an antitumor activity of the combination use of rIL-2, rIFN-αA/D and TMD-1 at a lower dosage level is measured.

Each drug was dissolved in a physiological saline solution (solvent) supplemented with 5% normal mouse serum and prepared in a form of a single preparation so that the dosage level is 0.1 mℓ/ mouse, calculated on the administration liquid volume basis. Each drug was intramuscularly administered at the left thigh once daily for 10 consecutive days from 7 days after tumor transplantation. The evaluation of antitumor effects was conducted by measuring tumor weight on the 21st day following the tumor transplantation, calculating the average tumor weight for each experimental group the results are shown in Table 5.

Table 5

| Drug | Dose Level rIFN-αA/D (units/mouse) | rIL-2 (mcg/mouse) | TMD-1 (mcg/mouse) | Number of Animals | Tumor Weight (g) Mean ± SD | Tumor Weight Ratio (T/C %) | Body Weight Gain (g) (7th day - 21st day) |
|---|---|---|---|---|---|---|---|
| Non-treated control | | | | 10 | 5.75 ± 1.86 | | |
| Solvent (control) | | | | 5 | 5.75 ± 0.43 | 100 | 4.3 |
| rIFN-αA/D | $1 \times 10^4$ | | | 5 | 4.84 ± 1.25 | 84 | 3.6 |
| rIL-2 | | 1 | | 5 | 3.61 ± 1.15 | 63 | 2.0 |
| TMD-1 | | | 100 | 5 | 4.43 ± 0.66 | 77 | 3.1 |
| rIFN-αA/D + rIL-2 | $1 \times 10^4$ | 1 | | 5 | 2.48 ± 1.30 | 43 | 0.1 |
| rIFN-αA/D + TMD-1 | $1 \times 10^4$ | | 100 | 5 | 4.68 ± 0.68 | 82 | 3.0 |
| rIL-2 + TMD-1 | | 1 | 100 | 5 | 2.34 ± 0.64 | 41 | 1.8 |
| rIFN-αA/D + rIL-2 + TMD-1 | $1 \times 10^4$ | 1 | 50 | 5 | 1.33 ± 0.84 | 23 | 1.1 |
| | $1 \times 10^4$ | 1 | 100 | 5 | 0.75 ± 0.73 | 13 | 0.2 |

Experimental Example 4

Antitumor Action in Intramuscular Administration of rIL-2 in combination with rIFN-αA/D and TMD-1:

Using injecting cylinders, 0.1 mℓ of tumor homogenate of Colon carcinoma 26 which was prepared by mixing 1 g of the tumor with 4 mℓ of physiological saline and by homogenizing the mixture using a Potter homogenizer was subcutaneously transplanted to the flank of female BALB/c mice weighing about 20 g. On the 7th day following the tumor transplantation, mice having a tumor reaching the specified level in size were selected and grouped, and drug administration was initiated. Each drug was intramuscularly administered at the left thigh once daily for 10 consecutive days. Each drug was dissolved in a physiological saline solution (solvent) supplemented with 5% normal mouse serum and prepared in a form of a single preparation so that the dosage level is 0.1 mℓ/mouse, calculated on the administration liquid volume basis. The evaluation of antitumor effects was conducted by measuring tumor weight on the 21st day following the tumor transplantation, calculating the average tumor weight for each experimental group, and calculating the tumor weight ratio (T/C %) of each drug-treated group (T, 5 mice in each group) to the non-treated control group (C, 5 to 10 mice in the group). Daily dosage levels are shown in either drug weight (mcg) or interferon activity (unit) per mouse. The results of administrations of IL-2 alone IFN-α alone, TMD-1 alone, and the antitumor agent comprising a combination of IL-2, IFN-α and TMD-1 of the present invention are shown in Table 6.

Table 6

| Drug | Dose Level rIFN-αA/D (units/mouse) | rIL-2 (mcg/mouse) | TMD-1 (mcg/mouse) | Number of Animals | Tumor Weight (g) Mean ± SD | Tumor Weight Ratio (T/C %) | Body Weight Gain (g) (7th day - 21st day) |
|---|---|---|---|---|---|---|---|
| Non-treated control | | | | 10 | 1.62 ± 0.18 | | 1.0 |
| Solvent (control) | | | | 5 | 1.61 ± 0.16 | 99 | 0.4 |
| rIFN-αA/D | $1 \times 10^5$ | | | 5 | 0.86 ± 0.05 | 53 | -0.2 |
| rIL-2 | | 10 | | 5 | 0.82 ± 0.20 | 51 | 0.6 |
| TMD-1 | | | 200 | 5 | 1.38 ± 0.27 | 85 | 0.4 |
| rIFN-αA/D + rIL-2 | $1 \times 10^5$ | 10 | | 5 | 0.29 ± 0.07 | 18 | -1.6 |
| rIFN-αA/D + TMD-1 | $1 \times 10^5$ | | 200 | 5 | 0.64 ± 0.13 | 40 | -0.2 |
| rIL-2 + TMD-1 | | 10 | 200 | 5 | 0.67 ± 0.07 | 41 | 0.3 |
| rIFN-αA/D + rIL-2 + TMD-1 | $1 \times 10^5$ | 10 | 200 | 5 | 0.15 ± 0.03 | 10 | -2.2 |

Experimental Example 5

Antitumor Action in Intravenous, Subcutaneous and Intramuscular Administration of rIL-2 in combination with rIFN-αA/D and TMD-1:

Using injecting cylinders, 1 x $10^6$ Meth-A fibrosarcoma cells were subcutaneously transplanted to the flank of female BALB/c mice weighing about 20 g. On the 7th day following the tumor transplantation, mice having a tumor reaching the specified level in size were selected and grouped, and drug administration was intitiated. Each drug was intraveneously, subcutaneously or intramuscularly administered at the left thigh once daily for 10 consecutive days. Each drug was dissolved in a physiological saline solution (solvent) supplemented with 5% normal mouse serum and prepared in a form of a single preparation so that the dose level is 0.2 mℓ/mouse when intraveneously administered, or the dose level is 0.1 mℓ/mouse when subcutaneously or intramuscularly administered, calculated on the administration liquid volume basis. The evaluation of antitumor effects was conducted by measuring tumor weight on the 21st day following the tumor transplantation, calculating the average tumor weight for each experimental group, and calculating the tumor weight ratio (T/C %) of each drug-treated group (T, 5 mice in each group) to the non-treated control group (C, 10 mice in the group). Daily dose levels are shown in either drug weight (mcg) or interferon activity (unit) per mouse. The results of administrations of the present antitumor agent comprising a combination of rIL-2, rIFN-α and TMD-1 are shown in Table 7. As seen from the Table 7, the intramuscular administration brings a remarkably stronger antitumor effect.

Table 7

| Administration Route | Drag and Dose Level rIFN-αA/D (units/mouse) | rIL-2 (mcg/mouse) | TMD-1 (mcg/mouse) | Number of Animals | Tumor Weight (mg) Mean ± SD | Tumor Weight Ratio (T/C %) |
|---|---|---|---|---|---|---|
| | Non-treated control | | | 5 | 7.347 ± 0.835 | |
| Intravenous | $2 \times 10^4$ | 0.2 | 200 | 5 | 2.991 ± 1.321 | 41 |
| Subcutaneous | $2 \times 10^4$ | 0.2 | 200 | 5 | 2.960 ± 1.497 | 40 |
| Intramuscular | $2 \times 10^4$ | 0.2 | 200 | 5 | 0.707 ± 0.890 | 10 |

Example 1

One mℓ of an aqueous solution prepared by dissolving in distilled water for injection 525 units of IL-2 (IL-2 Ala species and IL-2 Met species), 3,750,000 units of interferon-αA, 5 mg HSA and a suitable amount of hydrochloric acid (for pH adjustment), previously filtered for bacterial removal, and as controls the equal amount of an aqueous solution containing no hydrochloric acid (for pH adjustment) and the equal amount of an aqueous solution containing neither hydrochloric acid (for pH adjustment) nor HSA are each pipetted into vials and lyophilized at -40°C, then the atmosphere in the vials is replaced with $N_2$ gas, and the vials are tightly stoppered.

Each of these lyophilized products is re-dissolved in 1 mℓ distilled water for injection immediately after preparation and after 1 month of storage at 40°C. The clarity and residual potency, calculated on the basis of the pre-lyophilization titer regarded as 100%, of these solutions are determined. The titration of IL-2 is conducted in accordance with the method described in European Patent Publication No. 158487; and the titration of interferon-αA, in accordance with the method described in Japanese Patent Laid-open No. 57-79897 which corresponds to European Patent Publication No. 43980. The results are as shown in Table 8.

Example 2

One mℓ of an aqueous solution of pH 4.2 obtained by dissolving in distilled water for injection 1,250 units of IL-2 (IL-2 Ala species), 11,250,000 units of interferon-αA, 5 mg HSA, 50 mg mannitol and a suitable amount of hydrochloric acid (for pH adjustment), previously filtered for bacterial removal, is pipetted into a vial and lyophilized in the same manner as in Example 1. The resulting lyophilized product is re-dissolved in the same manner as in Example 1 immediately after preparation and after 1 month of storage at 40°C. The clarity and residual potency of the resulting solutions is determined in the same manner as in Example 1. The results are shown in Table 9.

Table 8

| Mixing Agent | Immediately after Preparation | | | | After 1 Month of Storage at 40°C | | |
|---|---|---|---|---|---|---|---|
| | pH | Clarity | IL-2 Retention Ratio | Interferon-αA Retention Ratio | Clarity | IL-2 Retention Ratio | Interferon-αA Retention Ratio |
| HSA (5 mg) Hydrochloric acid | 4.5 | Clear | 98% | 97% | Clear | 99% | 95% |
| HSA (5 mg) | 6.8 | Turbidity noted | 85% | 96% | Turbidity noted | 71% | 94% |
| | 7.9 | Turbidity noted | 45% | 55% | Turbidity noted | 38% | 43% |

Table 9

| Immediately after Preparation | | | | After 1 Month of Storage at 40°C | | |
|---|---|---|---|---|---|---|
| Clarity | pH | IL-2 Retention Ratio | Interferon-αA Retention Ratio | Clarity | IL-2 Retention Ratio | Interferon-αA Retention Ratio |
| Clear | 4.3 | 101% | 98% | Clear | 97% | 95% |

Example 3

One mℓ of an aqueous solution of pH 4.2 obtained by dissolving in distilled water for injection 1,250 units of IL-2 (IL-2 Ala species), 11,250,000 units of interferon-αA, 5 mg HSA, 23 mg glycine and a suitable amount of hydrochloric acid (for pH adjustment), previously filtered for bacterial removal, is pipetted into a vial and lyophilized in the same manner as in Example 1. The resulting lyophilized product is re-dissolved in the same manner as in Example 1 immediately after preparation and after 1 month of storage at 40°C. The clarity and residual potency of the resulting solutions are determined in the same manner as in Example 1. The results are shown in Table 10.

Table 10

| Immediately after Preparation | | | | After 1 Month of Storage at 40°C | | |
|---|---|---|---|---|---|---|
| Clarity | pH | IL-2 Retention Ratio | Interferon-αA Retention Ratio | Clarity | IL-2 Retention Ratio | Interferon-αA Retention Ratio |
| Clear | 4.2 | 97% | 96% | Clear | 99% | 94% |

Example 4

One mℓ of an aqueous solution of pH 4.5 obtained by dissolving in distilled water for injection 1,250 units of IL-2 (IL-2 Ala species and IL-2 Met species), 11,250,000 units of interferon-αA, 5 mg HSA, 9 mg sodium chloride and a suitable amount of hydrochloric acid (for pH adjustment), previously filtered for bacterial removal, is pipetted into a vial and lyophilized in the same manner as in Example 1. The resulting lyophilized product is re-dissolved in the same manner as in Example 1 immediately after preparation and after 1 month of storage at 40°C. The clarity and residual potency of the resulting solutions are determined in the same manner as in Example 1. The results are shown in Table 11.

Table 11

| Immediately after Preparation | | | | After 1 Month of Storage at 40°C | | |
|---|---|---|---|---|---|---|
| Clarity | pH | IL-2 Retention Ratio | Interferon-αA Retention Ratio | Clarity | IL-2 Retention Ratio | Interferon-αA Retention Ratio |
| Clear | 4.5 | 94% | 96% | Clear | 92% | 95% |

Example 5

One mℓ of an aqueous solution of pH 4.2 obtained by dissolving in distilled water for injection 1,250 units of IL-2 (IL-2 Ala species and IL-2 Met species), 11,250,000 units of interferon-αA/D, 5 mg HSA, 50 mg mannitol and a suitable amount of hydrochloric acid (for pH adjustment), previously filtered for bacterial removal, is pipetted into a vial and lyophilized in the same manner as in Example 1. The resulting lyophilized product is re-dissolved in the same manner as in Example 1 immediately after preparation and after 1 month of storage at 40°C. The clarity and residual potency of the resulting solutions are determined in the same manner as in Example 1. The results are shown in Table 12.

Table 12

| Immediately after Preparation | | | | After 1 Month of Storage at 40°C | | |
|---|---|---|---|---|---|---|
| Clarity | pH | IL-2 Retention Ratio | Interferon-αA Retention Ratio | Clarity | IL-2 Retention Ratio | Interferon-αA Retention Ratio |
| Clear | 4.3 | 101% | 98% | Clear | 97% | 95% |

Example 6

One mℓ of an aqueous solution of pH 4.5 obtained by dissolving in distilled water for injection 1,250 units of IL-2 (IL-2 Ala species and IL-2 Met species), 11,250,000 units of interferon-αA, TMD-1 1 mg, 5 mg HSA, 9 mg sodium chloride and a suitable amount of hydrochloric acid( for pH adjustment), previously filtered for bacterial removal, is pipetted into a vial and lyophilized in the same manner as in Example 1. The resulting lyophilized product is re-dissolved in the same manner as in Example 1 immediately after preparation and after 1 month of storage at 40°C. The clarity and residual potency of the resulting solutions are determined in the same manner as in Example 1. The results are shown in Table 13.

Table 13

| Immediately after Preparation | | | | After 1 Month of Storage at 40°C | | |
|---|---|---|---|---|---|---|
| Clarity | pH | IL-2 Retention Ratio | Interferon-αA Retention Ratio | Clarity | IL-2 Retention Ratio | Interferon-αA Retention Ratio |
| Clear | 4.5 | 96% | 95% | Clear | 95% | 93% |

Example 7

One mℓ of an aqueous solution of pH 4.5 obtained by dissolving in distilled water for injection 1,250 units of IL-2 (IL-2 Ala species), 11,250,000 units of interferon-αA, TMD-1 1 mg, 5 mg HSA, 9 mg sodium chloride and a suitable amount of hydrochloric acid (for pH adjustment), previously filtered for bacterial removal, is pipetted into a vial and lyophilized in the same manner as in Example 1. The resulting lyophilized product is re-dissolved in the same manner as in Example 1 immediately after preparation and after 1 month of storage at 40°C. The clarity and residual potency of the resulting solutions are determined in the same manner as in Example 1. The results are shown in Table 14.

Table 14

| Immediately after Preparation | | | | After 1 Month of Storage at 40°C | | |
|---|---|---|---|---|---|---|
| Clarity | pH | IL-2 Retention Ratio | Interferon-αA Retention Ratio | Clarity | IL-2 Retention Ratio | Interferon-αA Retention Ratio |
| Clear | 4.5 | 97% | 96% | Clear | 96% | 94% |

**Claims**

1. Use of an interleukin-2 active substance in combination with an interferon-α active substance for the production of a pharmaceutical preparation, wherein the administration route is intramuscular injection, for treating or improving a tumor disease of a warm-blooded animal.

2. A use as claimed in Claim 1, wherein a muramyldipeptide is further employed.

3 A use as claimed in Claim 1 or 2, wherein the compounds are used in a form of a single preparation.

4. A use as claimed in Claim 1 or 2, wherein the compounds are separately used.

5. A use as claimed in Claim 1, wherein the interleukin-2 active substance is a recombinant non-glycosylated human interleukin-2.

6. A use as claimed in Claim 1, wherein the interferon-α active substance is recombinant interferon-α.

7. A use as claimed in Claim 2, wherein the muramyldipeptide is a compound of the formula:

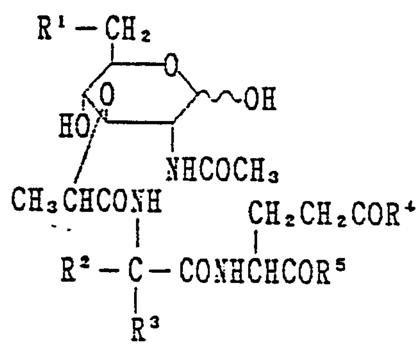

wherein $R^1$ is hydroxyl group or a carboxylic acid residue of the formula:

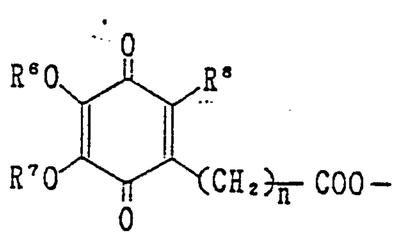

wherein $R^6$, $R^7$ and $R^8$ each dependently is a $C_{1-4}$ alkyl and n is an integer of 1 to 10, $R^2$ and $R^3$ is each independently hydrogen or a $C_{1-6}$ alkyl group which may optionally be substituted by a hydroxyl group, $R^4$ is a hydroxyl group or a $C_{1-6}$ alkoxy group, and $R^5$ is a hydroxyl group or an amino group.

8. An antitumor agent which comprises an interleukin-2 active substance in combination with an interferon-α active substance, wherein the administration route is intramuscular injection.

9. An antitumor agent as claimed in Claim 8, wherein the agent further comprises a muramyldipeptide.

10. An agent as claimed in Claim 8, wherein the interleukin-2 active substance is a recombinant non-glycosylated human interleukin-2.

11. An agent as claimed in Claim 8, wherein the interferon-α active substance is recombinant interferon-α.

12. An agent as claimed in Claim 9, wherein the muramyldipeptide is a compound of the formula:

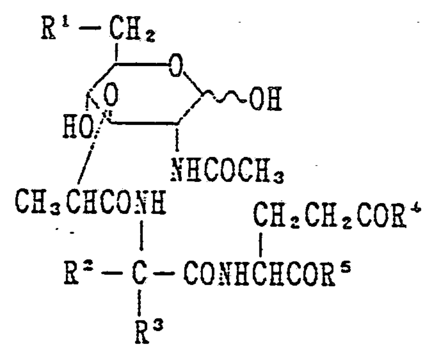

wherein $R^1$ is hydroxyl group or a carboxylic acid residue of the formula:

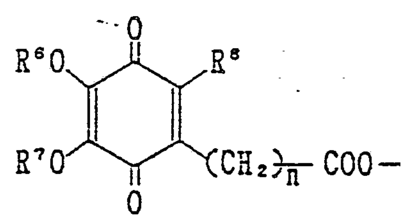

wherein $R^6$, $R^7$ and $R^8$ each dependently is a $C_{1-4}$ alkyl and n is an integer of 1 to 10, $R^2$ and $R^3$ is each independently hydrogen or a $C_{1-6}$ alkyl group which may optionally be substituted by a hydroxyl group, $R^4$ is a hydroxyl group or a $C_{1-6}$ alkoxy group, and $R^5$ is a hydroxyl group or an amino group.

Fig. 1

1
Ala Pro Thr Ser Ser Ser Thr Lys Lys Thr Gln Leu Gln

20
Leu Glu His Leu Leu Leu Asp Leu Gln Met Ile Leu Asn

Gly Ile Asn Asn Tyr Lys Asn Pro Lys Leu Thr Arg Met

40
Leu Thr Phe Lys Phe Tyr Met Pro Lys Lys Ala Thr Glu

60
Leu Lys His Leu Gln Cys Leu Glu Glu Glu Leu Lys Pro

Leu Glu Glu Val Leu Asn Leu Ala Gln Ser Lys Asn Phe

80
His Leu Arg Pro Arg Asp Leu Ile Ser Asn Ile Asn Val

100
Ile Val Leu Glu Leu Lys Gly Ser Glu Thr Thr Phe Met

Cys Glu Tyr Ala Asp Glu Thr Ala Thr Ile Val Glu Phe

120
Leu Asn Arg Trp Ile Thr Phe Cys Gln Ser Ile Ile Ser

133
Thr Leu Thr